# EUROPEAN PATENT APPLICATION

(11) **EP 3 300 708 A1**
(43) Date of publication of application: **04.04.2018**
(21) Application number: 17192330.3
(22) Date of filing: 21.09.2017
(51) Int. Cl.: A61F 13/15, A61F 13/515, A61F 13/56

(54) **AN ABSORBENT SANITARY ARTICLE**

(30) Priority: 30.09.2016 IT 201600098504
(71) Applicant: Fameccanica.Data S.p.A., 65129 Pescara (IT)
(72) Inventor: GUALTIERI, Diego, I-65127 Pescara (IT)
(74) Representative: Marchitelli, Mauro

(57) **Abstract**

An absorbent sanitary article comprising a central body (12) including a topsheet (22), a backsheet (24) and an absorbent core (26) interposed between said topsheet (22) and said backsheet (24), said article (10) comprising at least one pair of side panels (16) extending laterally from side edges (14) of said central body (12), said side panels (16) having respective proximal edges (16a) fixed between respective side edges (22a, 24a) of said topsheet (22) and backsheet (24), wherein said proximal edges (16a) of said side panels (16) are fixed by means of first welds (27) to said topsheet (22) only and by means of second welds (28) both to said topsheet (22) and to said backsheet (24).

## Description

### Field of the invention

The present description refers to absorbent sanitary articles and specifically relates to an absorbent sanitary article comprising a central body and at least one pair of side panels projecting from opposite sides of said central body.

### Description of the prior art

A very widespread embodiment for producing absorbent sanitary articles, such as for example diapers for babies, training-pants, incontinence pads for adults, etc., envisages that the absorbent sanitary article presents a rectangular central body, capable of being arranged in the user's crotch region, and at least one pair of side panels projecting from the side edges of at least one end of the main body. The side panels are provided with closing formations that allow the absorbent sanitary article to close around the user's waist region.

The main body has a layered structure in which the following are usually recognizable (in addition to various other accessory elements):
- a top layer or "topsheet" permeable to body fluids, designed to face towards the user's body;
- a lower layer or "backsheet" impervious to body fluids, designed to face outwards, i.e. in the opposite position with respect to the user's body; and
- an absorbent core, interposed between the topsheet and the backsheet.

An absorbent sanitary article of this type is known, for example, from the document EP-B1-2285332 by the same Applicant, on which the preamble of claim 1 is based. This document describes an absorbent sanitary article having a central body comprising a topsheet, a backsheet, and an absorbent core interposed between the topsheet and the backsheet, and two pairs of side panels extending laterally from the central body, wherein said side panels have proximal edges arranged between the topsheet and the backsheet, connected by means of thermal welding or ultrasonic welding to the topsheet and by means of adhesive to the backsheet.

The technique used for fixing the side panels to the central body must ensure a strong connection of the panels and avoid detaching and/or tearing. On the other hand, the side panel fixing techniques must also meet production needs, for example, in terms of apparatus simplification and cost reduction. In particular, most recently, the use of adhesives is avoided or reduced as much as possible in the methods of producing absorbent sanitary products.

### Object and summary of the invention

The object of the invention is therefore to provide an absorbent sanitary article that is able to ensure a strong connection of the side panels to the central body and that completely eliminates - or substantially reduce - the use of adhesives for connecting the side panels.

According to the present invention, this object is achieved by an absorbent sanitary article having the characteristics forming the subject of claim 1.

The claims form an integral part of the technical disclosure provided in relation to the invention.

### Brief description of the attached drawings

The invention will now be described, purely by way of non-limiting example, with reference to the attached drawings, in which:
- Figure 1 is a plan schematic view of an absorbent sanitary article according to the present invention in an extended position,
- Figure 2 is a view on an enlarged scale of the part indicated by the arrow II in Figure 1, and
- Figure 3 is a schematic cross-section along the line III-III of Figure 2.

### Detailed description

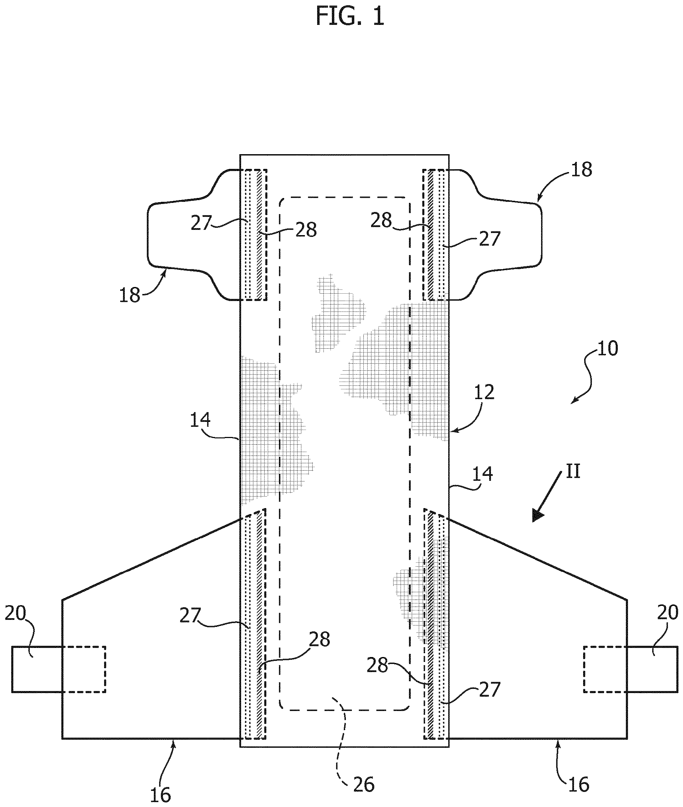

In Figure 1, the numerical reference 10 indicates an absorbent sanitary article according to the present invention, illustrated in a flat extended position. In the example illustrated in Figure 1, the absorbent sanitary article 10 is a diaper for babies, or incontinence pad for incontinent adults, intended to be sold open, and to be closed in the manner of pants after being arranged on the user's body. The solution described here, in any case, can also be applied to articles currently known as "training pants" intended to be sold already closed and to be worn by the users in the manner of pants.

The absorbent sanitary article 10 comprises a central body 12 having two side edges 14. In the example illustrated, the central body 12 has a rectangular shape and the two side edges 14 are straight and parallel to each other.

The absorbent sanitary article 10 comprises at least one pair of side panels 16. The side panels 16 extend outwardly from the side edges 14 of the central body 12 and allow the absorbent sanitary article 10 to be closed around the user's waist region by means of closing formations 20 (adhesive or with microhooks).

The side panels 16 are able to be manufactured according to criteria better described in the documents WO-A-01/91666 and WO-A-01/92013. The side panels 16 can be made of elastic material and can be provided with openings that give the side panels 16 breathability characteristics.

The side panels can be present at both ends (front and rear) of the central body 12. This is usually the case for articles of the "training pant" type, where the distal margins of the various panels are welded together, to give the article a closed conformation, when it is sold. It should be emphasized that the connotations "front" and "rear" are used here solely to distinguish between the two ends and therefore have no particular significance regarding the manner in which the product is finally worn.

The embodiment illustrated here refers to the case (most commonly in products sold in the "open" state) in which the side panels 16 are present at the rear end of the central body 12, while two wings 18 project laterally from the front end of the central body 12 giving the article 10 (seen in the open and extended position, as represented in Figure 1) a typical hourglass conformation.

The representation of Figure 1 is of schematic nature and intends to highlight that the solution described herein can be applied to a wide variety of possible constructive types of the article 10.

For a more detailed illustration of additional characteristics of the article 10 (for example, regarding the presence of contoured edges that delineate the contour of the user's leg openings, or for the presence of so-called "leg cuffs" with the function of lateral containment of body flows) reference can be made to the extensive literature that exists on this subject: this also applies in relation to the possible choice of materials constituting the various parts of the article 10.

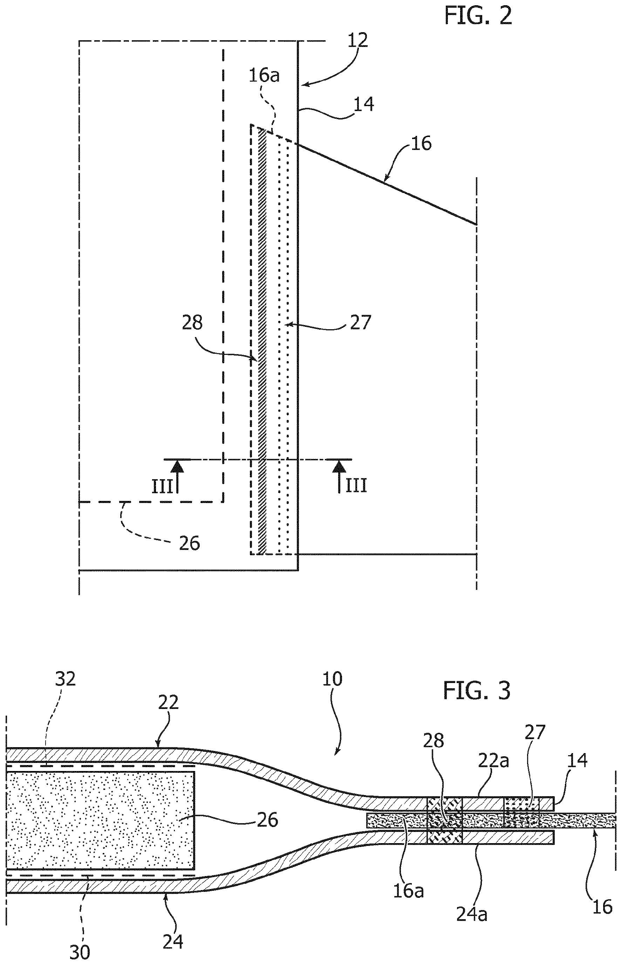

With reference to Figure 3, the central body 12 comprises (in addition to any accessory elements):
- a top layer or "topsheet" 22 permeable to body fluids, designed to be facing towards the user's body;
- a lower layer or "backsheet" 24 impervious to body fluids, designed to face outwards, i.e. in the opposite position with respect to the user's body; and
- an absorbent core 26, interposed between the topsheet 22 and the backsheet 24.

With reference to Figures 2 and 3, the solution for fixing the side panels 16 to the central body 10 according to the present invention envisages that the proximal edge of each side panel 16, indicated by 16a, is arranged in a sandwich-manner between corresponding side edges 22a, 24a of the topsheet 22 and of the backsheet 24. With reference to Figure 3, the proximal edge 16a of each side panel 16 is fixed by means of a first weld 27 to the topsheet 22 only and, by means of a second weld 28, it is fixed to both the topsheet 22 and the backsheet 24. The first weld 27 extends through the thickness of the side panel 16 and the topsheet 22, but does not affect the backsheet 24. The second weld 28 extends through the thickness of the topsheet 22, the side panel 16 and the backsheet 24, and fixes together the topsheet 22 and the backsheet 24 through the side panel 16.

With reference to Figure 2, the welds 27, 28 can be continuous or, preferably, discontinuous. The figures illustrate discontinuous welds formed by discrete welding points or dashes spaced apart in the longitudinal direction. The welds 27, 28 extend along lines parallel to the respective side edge 14 of the central body 12. Preferably, the first weld 27 is shifted outwardly with respect to the second weld 28.

The welds 27, 28 can be obtained by means of thermo-welding or ultrasonic welding, obtained with well-known devices in the field of producing absorbent sanitary articles.

In the production method, the side panels 16 are fixed at regular intervals on opposite sides of a continuous sheet of topsheets advancing in the machine direction. The first welds 27 that join the side panels 16 to the topsheet 22 are made prior to the application of the topsheet 22 to the backsheet 24, and have the object of keeping the side panels 16 joined to the topsheet 22 while it advances in the machine direction during the composition of a continuous chain of blanks of absorbent sanitary products.

The absorbent cores 26 are fixed to the backsheet 24 and/or to the topsheet 22, by welding or glue layers, indicated schematically by 30 and 32 in Figure 3, while the continuous sheets of topsheets 22 and backsheets 24 advance in the machine direction. After the two continuous sheets forming the topsheet 22 and the backsheet 24 are superimposed, the second welds 28 are carried out, which join together the topsheet 22, the backsheet 24 and the side panels 16.

The fixing technique described in relation to the rear side panels 16 also applies to the front wings 18 or any front side panels.

Tests carried out by the Applicant have shown that this connection mode is able to ensure a strong connection of the side panels 16 (and side wings 18) to the central body 12 of the absorbent sanitary article 10 without risk of accidental detaching or tearing, while at the same time minimizing the recourse of adhesive layers or glue.

The two welding lines 27, 28 cooperate with each other in order to maintain the three layers of the sandwich structure (topsheet 22, side panel 16 and backsheet 24) in a coplanar condition. In this way, it is possible to ensure that the proximal edge 16a of the side panel 16 remains aligned with the general placement plane of the topsheet 22, so that the strain stresses exerted on the side panel 16 during use of the article 10 are transmitted from the proximal edge 16a of the side panel 16 to the topsheet 22 merely as shear stresses acting in the common placement plane, i.e. in conditions where the second welding line 28 is able to exercise the maximum connecting and retention action.

Of course, without prejudice to the principle of the invention, the details of construction and the embodiments may vary, even significantly, with respect to those illustrated here, purely by way of non-limiting example, without departing from the scope of the invention as defined by the attached claims.

## Claims

1. An absorbent sanitary article comprising a central body (12) including a topsheet (22), a backsheet (24) and an absorbent core (26) interposed between said topsheet (22) and said backsheet (24), said article (10) comprising at least one pair of side panels (16) extending laterally from side edges (14) of said central body (12), said side panels (16) having respective proximal edges (16a) fixed between respective side edges (22a, 24a) of said topsheet (22) and backsheet (24), **characterized in that** said proximal edges (16a) of said side panels (16) are fixed by means of first welds (27) to said topsheet (22) only, and by means of second welds (28) both to said topsheet (22) and to said backsheet (24).

2. An absorbent sanitary article according to claim 1, wherein said first welds (27) and said second welds (28) extend along lines parallel to the respective side edges (14) of the central body (12).

3. An absorbent sanitary article according to claim 1 or claim 2, wherein said first welds (27) and said second welds (28) are discontinuous and each comprise discrete welding points or dashes spaced apart in the longitudinal direction.

4. An absorbent sanitary article according to any one of the preceding claims, wherein said first weld (27) is shifted outwardly with respect to said second weld (28).

5. An absorbent sanitary article according to any one of the preceding claims, wherein said first welds (27) and said second welds (28) are obtained by means of thermo-welding or ultrasonic welding.
